# EUROPÄISCHE PATENTSCHRIFT

(11) **EP 1 931 650 B1**
(45) Veröffentlichungstag und Bekanntmachung des Hinweises auf die Patenterteilung: **24.11.2010**
(21) Anmeldenummer: 06793286.3
(22) Anmeldetag: 06.09.2006
(51) Int. Cl.: C07D 317/12

(54) **VERFAHREN ZUR HERSTELLUNG VON DIOXOLAN**
METHOD FOR THE PRODUCTION OF DIOXOLANE
PROCEDE DE PRODUCTION DE DIOXOLANE

(30) Priorität: 07.09.2005 DE 102005042505
(43) Veröffentlichungstag der Anmeldung: 18.06.2008
(73) Patentinhaber: BASF SE, 67056 Ludwigshafen (DE)
(72) Erfinder: SIEGERT, Markus, 69126 Heidelberg (DE); LANG, Neven, 68163 Mannheim (DE); STRÖFER, Eckhard, 68163 Mannheim (DE); STAMMER, Achim, 67251 Freinsheim (DE)
(86) Internationale Anmeldenummer: PCT/EP2006/066086
(87) Internationale Veröffentlichungsnummer: WO 2007/028809

(56) Entgegenhaltungen:
- DE-B- 1 279 025
- JP-A- 5 271 217

## Beschreibung

Die Erfindung betrifft ein Verfahren zur Herstellung von Dioxolan.

1,3-Dioxacyclopentan, im Folgenden als Dioxolan bezeichnet, ist ein technisch verwendetes Derivat des Ethylenglykols, das sich durch Umsetzung von Ethylenglykol mit einer wässrigen Formaldehydlösung in Gegenwart von sauren Katalysatoren, wie Schwefelsäure, Bortrifluorid, Zinkchlorid oder sauren lonenaustauschern, herstellen lässt. Aus dem Reaktionsgemisch kann reines Dioxolan unter Einsatz unterschiedlicher Trennverfahren, insbesondere durch Destillation oder Extraktion, isoliert werden.

Dioxolan findet insbesondere als Comonomer bei der Polymerisation des Trioxans zu Polyoxymethylen-Copolymerisaten Verwendung.

Das am weitaus häufigsten eingesetzte Verfahren zur Zerlegung fluider Stoffgemische in Fraktionen oder reine Komponenten ist die Destillation bzw. Rektifikation. Die Trennwirkung dieses leistungsfähigen Verfahrens beruht auf dem Stoffaustausch zwischen einer gasförmigen und einer flüssigen Phase, die in unmittelbarer Berührung im Gegenstrom zueinander geführt werden.

Aus DE-A 1 279 025 ist ein Verfahren zur kontinuierlichen Reinigung eines Dioxolan enthaltenden Reaktionsgemisches aus der Umsetzung von Ethylenglykol mit Formaldehyd in Gegenwart von sauren Katalysatoren bekannt, wonach zunächst destillativ ein Roh-Dioxolan erhalten wird, das noch größere Mengen an Wasser, nicht umgesetztes Formaldehyd sowie kleinere Mengen an Säure und Alkohol, wie Ameisensäure und Methanol, enthält und das in Dampfform einer Destillationskolonne zugeführt wird, in der über Kopf ein azeotropes Gemisch von Dioxolan und Wasser abdestilliert wird, das maximal 10 Gew.-%, im Allgemeinen etwa 7 Gew.-% Wasser enthält. Das azeotrope Gemisch wird nach seiner Kühlung auf etwa 20 bis 40°C im Gegenstrom mit Alkalihydroxid und/oder einer konzentrierten wässrigen Alkalilauge behandelt, wodurch es weitgehend von Wasser und anderen Verunreinigungen befreit wird. Nach der Alkalibehandlung wird fraktioniert destilliert, unter Erhalt von gereinigtem Dioxolan, mit einem äußerst geringen Wassergehalt, von 50 ppm oder darunter, über den Kolonnensumpf. Das Verfahren hat den Nachteil, dass zum Erhalt von reinem Dioxolan mehrere Destillationskolonnen erforderlich sind, mit entsprechenden Investitions- und Betriebs-, insbesondere Energiekosten.

Aus der JP-A-5271217 ist ein analoges Verfahren bekannt, bei dem das Dioxolan aus dem Sumpf abgezogen wird.

Es war demgegenüber Aufgabe der Erfindung, ein einfacheres, insbesondere wirtschaftlicheres Verfahren zur Herstellung von reinem Dioxolan zur Verfügung zu stellen, das insbesondere in einer geringeren Zahl von Apparaten durchführbar ist, mit entsprechend niedrigeren Investitionskosten.

Die Erfindung besteht in einem Verfahren zur Herstellung von Dioxolan durch Umsetzung von Ethylenglykol mit Formaldehyd in wässriger Lösung in Gegenwart von Katalysatoren, das dadurch gekennzeichnet ist, dass die Umsetzung in einer Reaktivdestillationskolonne durchgeführt wird, wobei die Edukte Ethylenglykol und wässrige Formaldehydlösung der Reaktivdestillationskolonne im mittleren Bereich derselben zugeführt und ein Dioxolan enthaltender Strom mit mindestens 75 Gew.-% Dioxolan aus dem oberen Bereich der Reaktivdestillationskolonne sowie ein Sumpfstrom, enthaltend gegenüber Dioxolan schwersiedende Komponenten, abgezogen werden.

Es wurde gefunden, dass es möglich ist, die Umsetzung von Ethylenglykol und wässriger Formaldehydlösung in einer Reaktivdestillationskolonne durchzuführen und dabei aus dem oberen Bereich der Reaktivdestillationskolonne einen Strom mit einem hohen Anteil an Dioxolan, von mindestens 75 Gew.-% Dioxolan, zu erhalten.

Reaktivdestillationen sind bekannt und bezeichnen in bekannter Weise Verfahren, bei denen eine Reaktion und eine destillative Trennung stattfinden. Derartige Verfahren werden in Reaktivdestillationskolonnen durchgeführt, die mit trennwirksamen Einbauten ausgestattet sind, auf oder in die ein Katalysator auf- oder eingebracht ist. Hierbei kann es sich um Böden handeln, auf die eine Katalysatorschüttung aufgebracht ist, insbesondere aber um Packungen, wobei der Katalysator in die Packung eingebracht oder die Packung mit Katalysator beschichtet ist.

Im vorliegenden Verfahren werden die Edukte Ethylenglykol und Formaldehyd in wässriger Lösung, insbesondere voneinander getrennt, im mittleren Bereich einer Reaktivdestillationskolonne zugeführt, die mit reaktiven Einbauten versehen ist. In einer Ausführungsvariante werden die beiden Eduktströme der Reaktivdestillationskolonne auf gleiche Höhe zugeführt.

Als Katalysatoren eignen sich grundsätzlich alle bekannten Katalysatoren für die Umsetzung von Glykol mit wässriger Formaledehydlösung. Hierbei handelt es sich um saure Katalysatoren, wie Schwefelsäure, Bortrifluorid, Zinkchlorid oder saure lonenaustauscher.

Die Reaktivdestillationskolonne kann bei einem Kopfdruck im Bereich zwischen 0,01 bis 5 bar absolut, bevorzugt zwischen 0,15 und 2,50 bar absolut, besonders bevorzugt zwischen 0,20 und 1,50 bar absolut, betrieben werden. Bevorzugt wird sie mit 2 bis 75, insbesondere 5 bis 50, und besonders bevorzugt mit 10 bis 30 theoretischen Trennstufen ausgelegt.

Aus dem oberen Bereich der Reaktivdestillationskolonne wird ein Strom mit einem hohen Gehalt an Dioxolan, von mindestens 75 Gew.-%, bevorzugt von mindestens 80 Gew.-%, besonders bevorzugt von mindestens 85 Gew.-%, abgezogen. Das Verfahren kann insbesondere so geführt werden, dass aus dem oberen Bereich der Reaktivdestillationskolonne ein Gemisch mit der Zusammensetzung nahe dem binären Azeotrop Dioxolan/Wasser unter den Bedingungen des Kopfdruckes in der Reaktivdestillationskolonne und der entsprechenden Temperatur erhalten wird.

In dieser Ausführungsvariante wird das Gemisch mit der Zusammensetzung nahe der Zusammensetzung des binären Azeotrops Dioxolan/Wasser in einer nachfolgenden Druckwechselrektifikation zu Dioxolan aufgearbeitet: Hierzu wird das Azeotrop Dioxolan/Wasser einer ersten Destillationskolonne, im mittleren Bereich derselben, zugeführt, wobei diese erste Destillationskolonne bei einem Kopfdruck betrieben wird, der oberhalb des Kopfdruckes in der Reaktivdestillationskolonne liegt, insbesondere um mindestens 0,1 bar höher ist als der Kopfdruck in der Reaktivdestillationskolonne.

Aus der ersten Destillationskolonne wird ein Kopfstrom, enthaltend das binäre Azeotrop Dioxolan/Wasser unter den Bedingungen von Temperatur und Druck am Kopf der ersten Destillationskolonne abgezogen und aus dem Abtriebsteil der ersten Destillationskolonne, insbesondere aus dem Sumpf der ersten Destillationskolonne ein Strom, enthaltend Rein-Dioxolan.

Als Rein-Dioxolan wird vorliegend ein Strom verstanden, der zu mindestens 90 Gew.-%, insbesondere zu mindestens 95 Gew.-% oder auch zu mindestens 99 Gew.-% aus Dioxolan besteht.

Der Kopfstrom aus der ersten Destillationskolonne, enthaltend das binäre Azeotrop Dioxolan/Wasser unter den Bedingungen von Temperatur und Druck am Kopf der ersten Destillationskolonne wird in die Reaktivdestillationskolonne, bevorzugt in den oberen Teil derselben, recycliert.

Der Sumpfstrom aus der Reaktivdestillationskolonne, enthaltend gegenüber Dioxolan schwersiedende Komponenten, insbesondere Ethylenglykol und Wasser, wird bevorzugt einer zweiten Destillationskolonne zugeführt, die insbesondere bei einem Kopfdruck im Bereich zwischen 0,01 und 5,00 bar absolut, weiter bevorzugt zwischen 0,15 und 2,50 bar absolut oder auch zwischen 0,20 und 1,50 bar absolut betrieben wird, und aus der ein wasserreicher Kopfstrom, mit einem Wassergehalt von größer als 75 Gew.-%, bevorzugt größer als 90 Gew.-%, besonders bevorzugt größer als 99 Gew.-% abgezogen wird sowie ein ethylenglykolreicher Sumpfstrom, enthaltend mindestens 90 Gew.-% Ethylenglykol.

Um zu vermeiden, dass sich Schwersieder in der Anlage aufpegeln, wird bevorzugt ein kleiner Teilstrom des Sumpfstromes ausgeschleust. Der Sumpfstrom wird im Übrigen in die Reaktivdestillationskolonne recycliert. Bevorzugt wird er mit dem Ethylenglykolstrom vermischt und der Reaktivdestillationskolonne zugeführt.

In einer besonders vorteilhaften Verfahrensvariante ist die Reaktivdestillationskolonne als Trennwandkolonne ausgebildet. Trennwandkolonnen sind in bekannter Weise Kolonnen, in denen durch Einbau einer Trennwand eine Quervermischung von Flüssigkeits- und Brüdenströmen in Teilbereichen derselben verhindert wird.

Die Trennwandkolonne weist eine in Kolonnenlängsrichtung angeordnete Trennwand auf, die den Kolonneninnenraum in einen Zuführbereich, einen Entnahmebereich, einen unteren gemeinsamen Kolonnenbereich sowie einen oberen gemeinsamen Kolonnenbereich aufteilt.

In der Trennwandkolonne sind trennwirksame Einbauten eingebracht, insbesondere Packungen oder Böden sowie der Katalysator für die Umsetzung zu Dioxolan, der auf und/oder in den Einbauten eingebracht sein kann.

Der Trennwandkolonne werden die Edukte Ethylenglykol und wässrige Formaldehydlösung im Zuführbereich derselben, z.B. auf gleicher Höhe, zugeführt. In einer Verfahrensvariante wird Ethylenglykol oberhalb der wässrigen Formaldehydlösung in den Zuführbereich der Trennwandkolonne eingeleitet.

Aus dem oberen Bereich, insbesondere vom Kopf der Trennwandkolonne wird ein Dioxolan enthaltender Strom abgezogen, mit einem Mindestanteil an Dioxolan von 75 Gew.-%. Aus dem Kolonnensumpf wird ein Strom abgezogen, enthaltend gegenüber Dioxolan schwersiedende Komponenten, insbesondere Ethylenglykol.

Aus dem Entnahmebereich der Trennwandkolonne wird ein wasserreicher Strom abgezogen, mit einem Wassergehalt von größer als 75 Gew.-%, bevorzugt größer als 90 Gew.-%, besonders bevorzugt größer als 99 Gew.-%, der dem behandlungsbedürftigen Abwasser zugeführt wird.

In einer Verfahrensvariante wird aus der Trennwandkolonne ein Kopfstrom mit der Zusammensetzung des binären Azeotrops Dioxolan/Wasser abgezogen, einer ersten Destillationskolonne zugeführt, deren Kopfdruck höher ist als der Kopfdruck in der Trennwandkolonne, insbesondere um 0,1 bar und die insbesondere mit einer theoretischen Trennstufenzahl zwischen 2 und 75, bevorzugt zwischen 5 und 50, ausgelegt ist.

Aus der ersten Destillationskolonne wird ein Kopfstrom, enthaltend das binäre Azeotrop Dioxolan/Wasser unter den Bedingungen von Temperatur und Druck am Kopf der ersten Destillationskolonne abgezogen und aus dem Sumpf ein Strom, enthaltend Rein-Dioxolan, das der vorstehend angegebenen Definition entspricht.

In einer besonders vorteilhaften Verfahrensvariante wird eine spezielle Zuführung der Edukte Ethylenglykol und wässrige Formaldehydlösung gewählt, und zwar wird das Ethylenglykol erstens oberhalb der wässrigen Formaldehydlösung und zweitens in einem molaren Überschuss gegenüber Ethylenglykol, und zwar so, dass das Flüssigkeitsgemisch in der Kolonne einen Gehalt an Ethylenglykol von größer als 25 Gew.-% aufweist:

Die Erfinder haben erkannt, dass sich das binäre Dampf-Flüssigkeits-Phasengleichgewicht durch die Anwesenheit von Ethylenglykol derart verändert, dass das Azeotrop Dioxolan/Wasser gänzlich verschwindet.

Sofern der Anteil an gegenüber Dioxolan leichtsiedenden und/oder schwersiedenden Komponenten im Kopfstrom der Reaktivdestillationskolonne noch zu hoch ist, ist es möglich, denselben einer ersten Destillationskolonne zuzuführen, und darin in einen Kopfstrom, enthaltend gegenüber Dioxolan leichter siedende Komponenten, gegebenenfalls einen Sumpfstrom, enthaltend gegenüber Dioxolan schwersiedende Komponenten sowie einen Rein-Dioxolanstrom aus dem unteren Bereich des Abtriebsteils aufzutrennen. Sofern der Anteil an Schwersieder die Spezifikationsanforderungen nicht überschreitet, ist es auch möglich, den Rein-Dioxolanstrom als Sumpfstrom aus der ersten Destillationskolonne abzuziehen.

Der Sumpfstrom aus der Reaktivdestillationskolonne wird, wie in vorstehend beschriebenen Verfahrensvarianten, einer zweiten Destillationskolonne zugeführt und darin in eine wasserreiche Kopffraktion sowie eine ethylenglykolreiche Sumpffraktion aufgetrennt, die bevorzugt, bis auf einen kleinen Teilstrom, der ausgeschleust wird, in die Reaktivdestillationskolonne, bevorzugt in den Ethylenglykol/Feedstrom, recycliert wird.

In einer weiteren Variante kann das Verfahren ohne Einsatz der ersten Destillationskolonne in der Weise betrieben werden, dass am Kopf der Reaktivdestillationskolonne ein Strom, enthaltend gegenüber Dioxolan leichtsiedende Komponenten sowie aus dem oberen Bereich der Reaktivdestillationskolonne, unterhalb des Kopfstroms, ein Strom enthaltend Rein-Dioxolan abgezogen werden.

In einer besonders vorteilhaften Variante kann das Verfahren mit der oben angegebenen speziellen Zuführung der Eduktströme, das heißt mit Zuführung des Ethylenglykols oberhalb der wässrigen Formaldehydlösung sowie einem molaren Ethylenglykolüberschuss gegenüber Formaldehyd, wobei die Reaktivdestillationskolonne als Trennwandkolonne ausgestaltet ist, so durchgeführt werden, dass das Flüssigkeitsgemisch in der Kolonne einen Gehalt an Ethylenglykol von größer als 20 Gew.-% aufweist.

In dieser Verfahrensvariante wird der Ethylenglykol-Feedstrom oberhalb der wässrigen Formaldehydlösung in den Zuführbereich der Trennwandkolonne, besonders bevorzugt am oberen Ende desselben eingeleitet, und die wässrige Formaldehydlösung bevorzugt im mittleren Bereich des Zuführbereichs der Trennwandkolonne.

Aus dem oberen gemeinsamen Kolonnenbereich wird ein Kopfstrom, enthaltend gegenüber Dioxolan leichtsiedende Komponenten sowie unterhalb desselben, ebenfalls aus dem oberen gemeinsamen Kolonnenbereich Rein-Dioxolan abgezogen.

Der Sumpfstrom, enthaltend gegenüber Dioxolan schwersiedende Komponenten, wird bevorzugt, bis auf einen kleinen Teilstrom von der ausgeschleust wird, in die als Trennwandkolonne ausgebildete Reaktivdestillationskolonne, insbesondere in den Ethylenglykol-Feedstrom, recycliert.

Aus dem Entnahmebereich der Trennwandkolonne wird ein wasserreicher Strom, insbesondere mit einem Wasseranteil von größer als 75 Gew.-%, bevorzugt größer als 90 Gew.-%, besonders bevorzugt größer als 99 Gew.-% von einer Trennstufe abgezogen, die insbesondere auf gleiche Höhe oder unterhalb der Zuführung der wässrigen Formaldehydlösung liegt.

Die Erfindung wird im Folgenden anhand von Ausführungsbeispielen sowie einer Zeichnung näher erläutert.

Es zeigen im Einzelnen:
- Figur 1: die schematische Darstellung an Anlage für eine Verfahrensvariante,
- Figuren 2 bis 5: jeweils schematische Darstellungen von Anlagen für weitere bevorzugte Ver- fahrensvarianten.

In den Figuren bezeichnen gleiche Bezugszeichen jeweils gleiche oder entsprechende Merkmale.

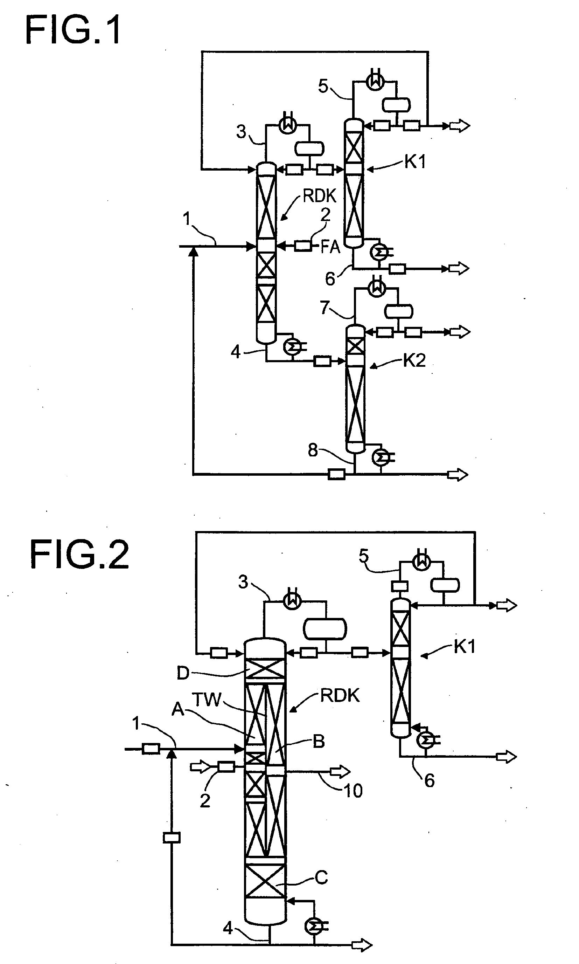

In dem in Figur 1 dargestellten Verfahrensschema wird ein Eduktstrom 1, enthaltend Ethylenglykol und ein Eduktstrom 2, enthaltend eine wässrige Formaldehydlösung einer Reaktivdestillationskolonne RDK zugeführt, die trennwirksame Einbauten und, zumindest in Teilbereichen derselben, reaktive Einbauten aufweist. Aus der Reaktivdestillationskolonne wird ein Kopfstrom 3, enthaltend das Azeotrop Dioxolan/Wasser abgezogen, in einem Kondensator am Kolonnenkopf kondensiert, teilweise als Rücklauf wieder auf die Reaktivdestillationskolonne aufgegeben und im Übrigen einer ersten Destillationskolonne K1 zugeführt, die bei höherem Kopfdruck als die Reaktivdestillationskolonne RDK betrieben wird, und darin in einen Kopfstrom, enthaltend das Azeotrop Dioxolan/Wasser beim Kopfdruck der ersten Destillationskolonne K1 aufgeteilt, der in einem Kondensator kondensiert, teilweise in die Reaktivdestillationskolonne RDK, im oberen Bereich derselben, recycliert und im Übrigen ausgeschleust. Aus der ersten Destillationskolonne wird ein Sumpfstrom 6, enthaltend Rein-Dioxolan, abgezogen.

Der Sumpfstrom 4 aus der Reaktivdestillationskolonne RDK enthält gegenüber Dioxolan schwersiedende Komponenten, insbesondere Ethylenglykol und Wasser. Der Sumpfstrom 4 aus der Reaktivdestillationskolonne RDK wird einer zweiten Destillationskolonne, K2, zugeführt und darin in einen wasserreichen Kopfstrom 7, und einen Sumpfstrom 8, enthaltend überwiegend Glykol, aufgetrennt. In der in der Figur dargestellten bevorzugten Verfahrensvariante wird ein Teilstrom des Sumpfstromes 8 ausgeschleust und der Sumpfstrom 8 im Übrigen in die Reaktivdestillationskolonne, in den Ethylenglykol-Feedstrom 1, recycliert.

Die in Figur 2 dargestellte Verfahrensvariante zeigt eine als Trennwandkolonne ausgebildete Reaktivdestillationskolonne RDK mit in Kolonnenlängsrichtung angeordneter Trennwand TW, die den Innenraum der Reaktivdestillationskolonne RDK in einen Zuführbereich A, einen Entnahmebereich B, einen unteren gemeinsamen Kolonnenbereich C sowie einen oberen gemeinsamen Kolonnenbereich D aufteilt.

Die Feedströme Ethylenglykol (Strom 1) und wässrige Formaldehydlösung (Strom 2) werden in den Zuführbereich eingeleitet, in der in der Figur dargestellten bevorzugten Verfahrensvariante Strom 1 oberhalb von Strom 2. Aus dem Entnahmeteil B wird ein wasserreicher Strom 10 abgezogen, der dem behandlungsbedürftigen Abwasser zugeführt wird. Aus dem oberen gemeinsamen Kolonnenbereich D wird ein Strom 3, enthaltend das Azeotrop Dioxolan/Wasser abgezogen, in einem Kondensator am Kolonnenkopf kondensiert, teilweise als Rücklauf wieder auf die Reaktivdestillationskolonne RDK aufgegeben und im Übrigen einer ersten Destillationskolonne K1 zugeführt, die bei höherem Kopfdruck gegenüber der als Trennwandkolonne ausgebildeten Reaktivdestillationskolonne betrieben wird, und darin in einen Kopfstrom 5 aufgetrennt, enthaltend das Azeotrop Dioxolan/Wasser unter den Bedingungen von Temperatur und Druck am Kopf der ersten Destillationskolonne K1, der teilweise abgezogen und im Übrigen in die Reaktivdestillationskolonne RDK, im oberen Bereich derselben recycliert wird, sowie einen Sumpfstrom 6, enthaltend Rein-Dioxolan.

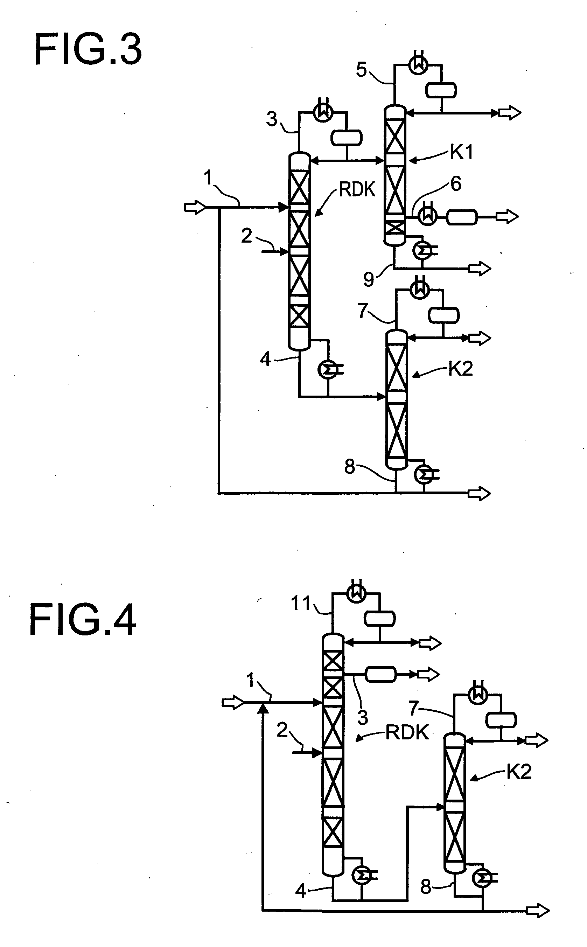

Die in Figur 3 dargestellte Verfahrensvariante zeigt eine Zuführung des Ethylenglykol-Feedstroms 1 oberhalb des Stromes 2 aus wässriger Formaldehydlösung.

Der Kopfstrom 3 aus der Reaktivdestillationskolonne RDK wird einer ersten Destillationskolonne K1 zugeführt und darin in einen Kopfstrom 5, enthaltend Leichtsieder, den Sumpfstrom 9 sowie einen Strom 6 aus dem unteren Bereich des Abtriebsteils, enthaltend Rein-Dioxolan, aufgetrennt.

Der Sumpfstrom 4 aus der Reaktivdestillationskolonne RDK wird einer zweiten Destillationskolonne K2 zugeführt und darin in einen wasserreichen Kopfstrom 7 sowie einen Sumpfstrom 8, enthaltend überwiegend Ethylenglykol aufgetrennt, der, in der in Figur 2 dargestellten bevorzugten Ausführungsvariante teilweise ausgeschleust und im Übrigen in die Reaktivdestillationskolonne RDK, in den Ethylenglykol-Feedstrom 1, recycliert wird.

Figur 4 zeigt eine Variante des in Figur 3 dargestellten Verfahrens ohne erste Destillationskolonne K1; aus der Reaktivdestillationskolonne RDK wird ein Kopfstrom 11, enthaltend Leichtsieder sowie unterhalb desselben, aus dem oberen Bereich der Reaktivdestillationskolonne RDK, ein Rein-Dioxolan enthaltender Strom 3 abgezogen.

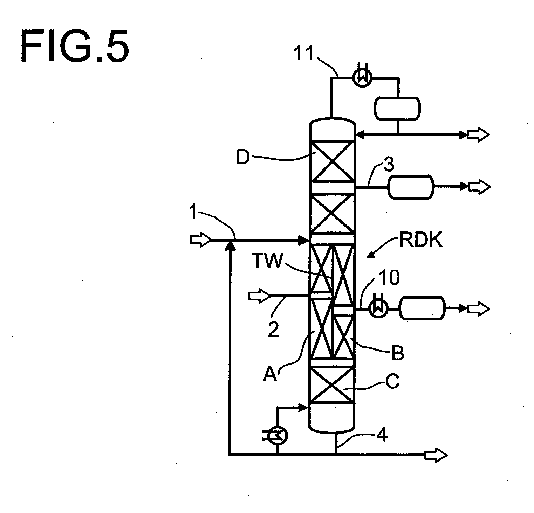

Figur 5 zeigt eine besonders bevorzugte Verfahrensvariante mit einer einzigen Kolonne, und zwar der Reaktivdestillationskolonne RDK, die als Trennwandkolonne mit in Kolonnenlängsrichtung angeordneter Trennwand TW ausgebildet ist. Der Ethylenglykol-Feedstrom 1 wird im oberen Teil des Zuführbereichs A und die wässrige Formaldehydlösung, Strom 2, unterhalb desselben, im mittleren Teil des Zuführbereichs A, eingeleitet. Aus dem Entnahmebereich wird ein wasserreicher Strom 10 abgezogen, aus dem oberen gemeinsamen Kolonnenbereich D ein Kopfstrom 11, enthaltend Leichtsieder sowie unterhalb desselben ein Strom 3, enthaltend Rein-Dioxolan.

Der Sumpfstrom 4, enthaltend überwiegend Ethylenglykol wird teilweise ausgeschleust und im Übrigen in die als Trennwandkolonne ausgebildete Reaktivdestillationskolonne, in den Ethylenglykol-Feedstrom 1, recycliert.

## Patentansprüche

1. Verfahren zur Herstellung von Dioxolan durch Umsetzung von Ethylenglykol (1) mit Formaldehyd in wässriger Lösung (2) in Gegenwart von Katalysatoren, **dadurch gekennzeichnet, dass** die Umsetzung in einer Reaktivdestillationskolonne (RDK) durchgeführt wird, wobei die Edukte Ethylenglykol (1) und wässrige Formaldehydlösung (2) der Reaktivdestillationskolonne (RDK) im mittleren Bereich derselben zugeführt und ein Dioxolan enthaltender Strom (3) mit mindestens 75 Gew.-% Dioxolan aus dem oberen Bereich der Reaktivdestillationskolonne (RDK) sowie ein Sumpfstrom (4), enthaltend gegenüber Dioxolan schwersiedende Komponenten, abgezogen werden.

2. Verfahren nach Anspruch 1, **dadurch gekennzeichnet, dass** die Reaktivdestillationskolonne (RDK) mit einem Kopfdruck im Bereich von 0,01 bis 50 bar absolut, bevorzugt von 0,15 bis 2,50 bar absolut, besonders bevorzugt von 0,2 bis 1,50 bar absolut, betrieben wird.

3. Verfahren nach Anspruch 1 oder 2, **dadurch gekennzeichnet, dass** der Dioxolan enthaltende Strom (3) aus dem oberen Bereich der Reaktivdestillationskolonne (RDK) die Zusammensetzung des Azeotrops Dioxolan/Wasser unter den Bedingungen von Temperatur und Druck im oberen Bereich der Reaktivdestillationskolonne aufweist, dass der Dioxolan enthaltende Strom (3) einer ersten Destillationskolonne (K1) zugeführt wird, deren Kopfdruck oberhalb des Kopfdruckes der Reaktivdestillationskolonne (RDK) liegt und dass aus der ersten Destillationskolonne (K1) ein Kopfstrom (5) mit der Zusammensetzung des Azeotrops Dioxolan/Wasser unter den Bedingungen von Temperatur und Druck am Kopf der ersten Destillationskolonne (K1) abgezogen wird, der in die Reaktivdestillationskolonne (RDK) recycliert wird sowie ein Strom (6) aus dem Abtriebsteil der ersten Destillationskolonne (K1), insbesondere aus dem Sumpf der ersten Destillationskolonne (K1), enthaltend Rein-Dioxolan, mit einem Anteil an Dioxolan von größer als 90 Gew.-%, bevorzugt von größer als 95 Gew.-% und besonders bevorzugt von größer als 99 Gew.-%.

4. Verfahren nach einem der Ansprüche 1 bis 3, **dadurch gekennzeichnet, dass** der Sumpfstrom (4) aus der Reaktivdestillationskolonne (RDK), enthaltend gegenüber Dioxolan schwersiedende Komponenten, einer zweiten Destillationskolonne (K2) zugeführt wird, die bei einem Kopfdruck im Bereich von 0,1 bis 50 bar absolut, bevorzugt zwischen 0,15 und 2,50 bar absolut, besonders bevorzugt zwischen 0,2 und 1,50 bar absolut, betrieben wird, und aus der ein wasserreicher Kopfstrom (7), mit einem Wassergehalt von größer als 75 Gew.-%, bevorzugt von größer als 90 Gew.-%, besonders bevorzugt von größer als 99 Gew.-%, sowie ein ethylenglykolreicher Sumpfstrom (8) abgezogen werden.

5. Verfahren nach einem der Ansprüche 1 bis 4, **dadurch gekennzeichnet, dass** ein kleiner Teilstrom des ethylenglykolreichen Sumpfstromes (8) aus der zweiten Destillationskolonne (K2) ausgeschleust wird und der ethylenglykolreiche Sumpfstrom (8) im Übrigen in die Reaktivdestillationskolonne (RDK) recycliert wird.

6. Verfahren nach einem der Ansprüche 1 bis 3, **dadurch gekennzeichnet, dass** die Reaktivdestillationskolonne (RDK) als Trennwandkolonne ausgebildet ist, mit einer in Kolonnenlängsrichtung ausgerichteten Trennwand (TW), die den Innenraum der Reaktivdestillationskolonne (RDK) in einen Zuführbereich (A), einen Entnahmebereich (B), einen unteren gemeinsamen Kolonnenbereich (C) und einen oberen gemeinsamen Kolonnenbereich (D) aufteilt, und dass die Edukte Ethylenglykol (1) und wässrige Formaldehydlösung (2) dem Zuführbereich (A) zugeführt, der Strom (3) enthaltend Dioxolan, aus dem oberen gemeinsamen Kolonnenbereich (D) und der Sumpfstrom (4), enthaltend gegenüber Dioxolan schwersiedende Komponenten, aus dem unteren gemeinsamen Kolonnenbereich (C) sowie ein wasserreicher Strom (10), mit einem Wassergehalt von größer als 75 Gew.-%, bevorzugt von größer als 90 Gew.-%, besonders bevorzugt von größer als 99 Gew.-% aus dem Entnahmebereich (B) abgezogen werden.

7. Verfahren nach einem der Ansprüche 1 bis 6, **dadurch gekennzeichnet, dass** Ethylenglykol (1) gegenüber Formaldehyd in wässriger Lösung (2) in molarem Überschuss sowie oberhalb desselben der Reaktivdestillationskolonne (RDK) zugeführt wird.

8. Verfahren nach Anspruch 1 oder 2, **dadurch gekennzeichnet, dass** Ethylenglykol (1) gegenüber Formaldehyd und wässriger Lösung (3) in molarem Überschuss und oberhalb desselben der Reaktivdestillationskolonne (RDK) zugeführt wird und dass der Dioxolan enthaltende Strom (3) aus dem oberen Bereich der Reaktivdestillationskolonne (RDK) Rein-Dioxolan, mit einem Gehalt an Dioxolan von größer als 95 Gew.-%, bevorzugt von größer als 99 Gw.-%, besonders bevorzugt von größer als 99,5 Gew.-%, enthält.

9. Verfahren nach Anspruch 8, **dadurch gekennzeichnet, dass** aus der Reaktivdestillationskolonne (RDK) ein Kopfstrom 11, enthaltend gegenüber Dioxolan leichtsiedende Komponenten, abgezogen wird und dass der Sumpfstrom (4) aus der Reaktivdestillationskolonne (RDK) einer zweiten Destillationskolonne (K2) zugeführt wird, die bei einem Kopfdruck im Bereich von 0,1 bis 50 bar absolut, bevorzugt zwischen 0,15 und 2,50 bar absolut, besonders bevorzugt zwischen 0,2 und 1,50 bar absolut, betrieben wird, und aus der ein wasserreicher Kopfstrom (7), mit einem Wassergehalt von größer als 75 Gew.-%, bevorzugt von größer als 90 Gew.-%, besonders bevorzugt von größer als 99 Gew.-%, sowie ein ethylenglykolreicher Sumpfstrom (8) abgezogen werden.

10. Verfahren nach Anspruch 8, **dadurch gekennzeichnet, dass** die Reaktivdestillationskolonne (RDK) als Trennwandkolonne ausgebildet ist, mit einer in Längsrichtung angeordneten Trennwand (TW), die den Innenraum der Reaktivdestillationskolonne (RDK) in einen Zuführbereich (A), einen Entnahmebereich (B), einen unteren gemeinsamen Kolonnenbereich (C) und einen oberen gemeinsamen Kolonnenbereich (D) aufteilt, und dass die Edukte Ethylenglykol (1) und wässrige Formaldehydlösung (2) dem Zuführbereich (A) zugeführt, der Strom (3) enthaltend Dioxolan, aus dem oberen gemeinsamen Kolonnenbereich (D) und der Sumpfstrom (4), enthaltend gegenüber Dioxolan schwersiedende Komponenten, aus dem unteren gemeinsamen Kolonnenbereich (C) sowie ein wasserreicher Strom (10), mit einem Wassergehalt von größer als 75 Gew.-%, bevorzugt von größer als 90 Gew.-%, besonders bevorzugt von größer als 99 Ges.-% aus dem Entnahmebereich (B) abgezogen werden.

## Claims

1. A process for preparing dioxolane by reacting ethylene glycol (1) with formaldehyde in aqueous solution (2) in the presence of catalysts, wherein the reaction is carried out in a reactive distillation column (RDK), with the starting materials ethylene glycol (1) and aqueous formaldehyde solution (2) being fed into the reactive distillation column (RDK) in the middle region of the column and a dioxolane-comprising stream (3) comprising at least 75% by weight of dioxolane being taken off from the upper region of the reactive distillation column (RDK) and a bottom stream (4) comprising components having boiling points higher than that of dioxolane being taken off.

2. The process according to claim 1, wherein the reactive distillation column (RDK) is operated at a pressure at the top in the range from 0.01 to 50 bar absolute, preferably from 0.15 to 2.50 bar absolute, particularly preferably from 0.2 to 1.50 bar absolute.

3. The process according to claim 1 or 2, wherein the dioxolane-comprising stream (3) from the upper region of the reactive distillation column (RDK) has the composition of the azeotrope of dioxolane/water under the conditions of temperature and pressure in the upper region of the reactive distillation column, the dioxolane-comprising stream (3) is fed to a first distillation column (K1) in which the pressure at the top is above the pressure at the top of the reactive distillation column (RDK) and an overhead stream (5) having the composition of the azeotrope of dioxolane/water under the conditions of temperature and pressure at the top of the first distillation column (K1) is taken off from the first distillation column (K1) and is recycled to the reactive distillation column (RDK) and a stream (6) which comprises pure dioxolane and has a dioxolane content of greater than 90% by weight, preferably greater than 95% by weight and particularly preferably greater than 99% by weight, is taken off from the stripping section of the first distillation column (K1), in particular from the bottom of the first distillation column (K1).

4. The process according to any of claims 1 to 3, wherein the bottom stream (4) from the reactive distillation column (RDK), which comprises components having boiling points higher than that of dioxolane, is fed to a second distillation column (K2) which is operated at a pressure at the top in the range from 0.1 to 50 bar absolute, preferably from 0.15 to 2.50 bar absolute, particularly preferably from 0.2 to 1.50 bar absolute, and from which a water-rich overhead stream (7) having a water content of greater than 75% by weight, preferably greater than 90% by weight, particularly preferably greater than 99% by weight, and a bottom stream (8) which is rich in ethylene glycol are taken off.

5. The process according to any of claims 1 to 4, wherein a small substream of the bottom stream (8) which is rich in ethylene glycol from the second distillation column (K2) is discharged and the remainder of the bottom stream (8) which is rich in ethylene glycol is recycled to the reactive distillation column (RDK).

6. The process according to any of claims 1 to 3, wherein the reactive distillation column (RDK) is configured as a dividing wall column having a dividing wall (TW) which is aligned in the longitudinal direction of the column and divides the interior of the reactive distillation column (RDK) into a feed region (A), an offtake region (B), a lower combined column region (C) and an upper combined column region (D) and the starting materials ethylene glycol (1) and aqueous formaldehyde solution (2) are fed into the feed region (A), the stream (3) comprising dioxolane is taken off from the upper combined column region (D) and the bottom stream (4) comprising components having boiling points higher than that of dioxolane are taken off from the lower combined column region (C) and a water-rich stream (10) having a water content of greater than 75% by weight, preferably greater than 90% by weight, particularly preferably greater than 99% by weight, is taken off from the offtake region (B).

7. The process according to any of claims 1 to 6, wherein ethylene glycol (1) is fed into the reactive distillation column (RDK) in a molar excess over formaldehyde in aqueous solution (2) and at a point above this.

8. The process according to claim 1 or 2, wherein ethylene glycol (1) is fed into the reactive distillation column (RDK) in a molar excess over formaldehyde in aqueous solution (3) and at a point above this and the dioxolane-comprising stream (3) from the upper region of the reactive distillation column (RDK) comprises pure dioxolane having a dioxolane content of greater than 95% by weight, preferably greater than 99% by weight, particularly preferably greater than 99.5% by weight.

9. The process according to claim 8, wherein an overhead stream 11 comprising components having boiling points lower than that of dioxolane is taken off from the reactive distillation column (RDK) and the bottom stream (4) from the reactive distillation column (RDK) is fed to a second distillation column (K2) which is operated at a pressure at the top in the range from 0.1 to 50 bar absolute, preferably from 0.15 to 2.50 bar absolute, particularly preferably from 0.2 to 1.50 bar absolute, and from which a water-rich overhead stream (7) having a water content of greater than 75% by weight, preferably greater than 90% by weight, particularly preferably greater than 99% by weight, and a bottom stream (8) which is rich in ethylene glycol are taken off.

10. The process according to claim 8, wherein the reactive distillation column (RDK) is configured as a dividing wall column having a dividing wall (TW) which is aligned in the longitudinal direction and divides the interior of the reactive distillation column (RDK) into a feed region (A), an offtake region (B), a lower combined column region (C) and an upper combined column region (D) and the starting materials ethylene glycol (1) and aqueous formaldehyde solution (2) are fed into the feed region (A), the stream (3) comprising dioxolane is taken off from the upper combined column region (D) and the bottom stream (4) comprising components having boiling points higher than that of dioxolane is taken off from the lower combined column region (C) and a water-rich stream (10) having a water content of greater than 75% by weight, preferably greater than 90% by weight, particularly preferably greater than 99% by weight, is taken off from the offtake region (B).

## Revendications

1. Procédé de fabrication de dioxolane par réaction d'éthylène glycol (1) avec du formaldéhyde en solution aqueuse (2) en présence de catalyseurs, **caractérisé en ce que** la réaction est réalisée dans une colonne de distillation réactive (RDK), les réactifs éthylène glycol (1) et solution aqueuse de formaldéhyde (2) étant introduits dans la colonne de distillation réactive (RDK) dans la zone intermédiaire de celle-ci et un courant contenant du dioxolane (3) qui contient au moins 75 % en poids de dioxolane étant soutiré de la zone supérieure de la colonne de distillation réactive (RDK), ainsi qu'un courant de fond (4) contenant des composants de point d'ébullition élevé en comparaison du dioxolane.

2. Procédé selon la revendication 1, **caractérisé en ce que** la colonne de distillation réactive (RDK) est exploitée avec une pression de tête dans la plage allant de 0,01 à 50 bars absolus, de préférence de 0,15 à 2,50 bars absolus, de manière particulièrement préférée de 0,2 à 1,50 bars absolus.

3. Procédé selon la revendication 1 ou 2, **caractérisé en ce que** le courant contenant du dioxolane (3) issu de la zone supérieure de la colonne de distillation réactive (RDK) présente la composition de l'azéotrope dioxolane/eau dans les conditions de température et de pression de la zone supérieure de la colonne de distillation réactive, **en ce que** le courant contenant du dioxolane (3) est introduit dans une première colonne de distillation (K1), dont la pression de tête se situe au-dessus de la pression de tête de la colonne de distillation réactive (RDK), et **en ce qu'**un courant de tête (5) ayant la composition de l'azéotrope dioxolane/eau dans les conditions de température et de pression à la tête de la première colonne de distillation (K1) est soutiré de la première colonne de distillation (K1), et recyclé dans la colonne de distillation réactive (RDK), ainsi qu'un courant (6) issu de la zone de rectification de la première colonne de distillation (K1), notamment du fond de la première colonne de distillation (K1), contenant du dioxolane pur, ayant une proportion de dioxolane supérieure à 90 % en poids, de préférence supérieure à 95 % en poids et de manière particulièrement préférée supérieure à 99 % en poids.

4. Procédé selon l'une quelconque des revendications 1 à 3, **caractérisé en ce que** le courant de fond (4) de la colonne de distillation réactive (RDK), contenant des composants de point d'ébullition élevé en comparaison du dioxolane, est introduit dans une deuxième colonne de distillation (K2), qui est exploitée à une pression de tête dans la plage allant de 0,1 à 50 bars absolus, de préférence comprise entre 0,15 et 2,50 bars absolus, de manière particulièrement préférée comprise entre 0,2 et 1,50 bars absolus, et un courant de tête riche en eau (7), ayant une teneur en eau supérieure à 75 % en poids, de préférence supérieure à 90 % en poids, de manière particulièrement préférée supérieure à 99 % en poids, ainsi qu'un courant de fond riche en éthylène glycol (8), sont soutirés de celle-ci.

5. Procédé selon l'une quelconque des revendications 1 à 4, **caractérisé en ce qu'**un petit courant partiel du courant de fond riche en éthylène glycol (8) issu de la deuxième colonne de distillation (K2) est évacué et le reste du courant de fond riche en éthylène glycol (8) est recyclé dans la colonne de distillation réactive (RDK).

6. Procédé selon l'une quelconque des revendications 1 à 3, **caractérisé en ce que** la colonne de distillation réactive (RDK) est conçue sous la forme d'une colonne à paroi de séparation, avec une paroi de séparation (TW) dans la direction longitudinale de la colonne, qui partage l'espace intérieur de la colonne de distillation réactive (RDK) en une zone d'alimentation (A), une zone de soutirage (B), une zone de colonne commune inférieure (C) et une zone de colonne commune supérieure (D), et **en ce que** les réactifs éthylène glycol (1) et solution aqueuse de formaldéhyde (2) sont introduits dans la zone d'alimentation (A), le courant (3) contenant du dioxolane est soutiré à partir de la zone de colonne commune supérieure (D) et le courant de fond (4), contenant des composants de point d'ébullition élevé par rapport au dioxolane, à partir de la zone de colonne commune inférieure (C), ainsi qu'un courant riche en eau (10), ayant une teneur en eau supérieure à 75 % en poids, de préférence supérieure à 90 % en poids, de manière particulièrement préférée supérieure à 99 % en poids, à partir de la zone de soutirage (B).

7. Procédé selon l'une quelconque des revendications 1 à 6, **caractérisé en ce que** l'éthylène glycol (1) est introduit dans la colonne de distillation réactive (RDK) en excès molaire par rapport au formaldéhyde en solution aqueuse (2) et au-dessus de celui-ci.

8. Procédé selon la revendication 1 ou 2, **caractérisé en ce que** l'éthylène glycol (1) est introduit dans la colonne de distillation réactive (RDK) en excès molaire par rapport au formaldéhyde en solution aqueuse (2) et au-dessus de celui-ci, et **en ce que** le courant contenant du dioxolane (3) issu de la zone supérieure de la colonne de distillation réactive (RDK) contient du dioxolane pur, ayant une teneur en dioxolane supérieure à 95 % en poids, de préférence supérieure à 99 % en poids, de manière particulièrement préférée supérieure à 99,5 % en poids.

9. Procédé selon la revendication 8, **caractérisé en ce qu'**un courant de tête (11), contenant des composants de point d'ébullition bas en comparaison du dioxolane, est soutiré de la colonne de distillation réactive (RDK), et **en ce que** le courant de fond (4) issu de la colonne de distillation réactive (RDK) est introduit dans une deuxième colonne de distillation (K2), qui est exploitée à une pression de tête dans la plage allant de 0,1 à 50 bars absolus, de préférence comprise entre 0,15 et 2,50 bars absolus, de manière particulièrement préférée comprise entre 0,2 et 1,50 bars absolus, et un courant de tête riche en eau (7), ayant une teneur en eau supérieure à 75 % en poids, de préférence supérieure à 90 % en poids, de manière particulièrement préférée supérieure à 99 % en poids, ainsi qu'un courant de fond riche en éthylène glycol (8), sont soutirés de celle-ci.

10. Procédé selon la revendication 8, **caractérisé en ce que** la colonne de distillation réactive (RDK) est conçue sous la forme d'une colonne à paroi de séparation, avec une paroi de séparation (TW) dans la direction longitudinale, qui partage l'espace intérieur de la colonne de distillation réactive (RDK) en une zone d'alimentation (A), une zone de soutirage (B), une zone de colonne commune inférieure (C) et une zone de colonne commune supérieure (D), et **en ce que** les réactifs éthylène glycol (1) et solution aqueuse de formaldéhyde (2) sont introduits dans la zone d'alimentation (A), le courant (3) contenant du dioxolane est soutiré à partir de la zone de colonne commune supérieure (D) et le courant de fond (4), contenant des composants de point d'ébullition élevé par rapport au dioxolane, à partir de la zone de colonne commune inférieure (C), ainsi qu'un courant riche en eau (10), ayant une teneur en eau supérieure à 75 % en poids, de préférence supérieure à 90 % en poids, de manière particulièrement préférée supérieure à 99 % en poids, à partir de la zone de soutirage (B).
